# EUROPEAN PATENT SPECIFICATION

(11) **EP 3 970 641 B1**
(45) Date of publication and mention of the grant of the patent: **23.04.2025**
(21) Application number: 21195117.3
(22) Date of filing: 06.09.2021
(51) Int. Cl.: A61B 17/22, A61B 1/018, A61B 1/307

(54) **ENDOSCOPIC TIP EXTENDER**
ENDOSKOPIESPITZENVERLÄNGERER
EXTENSION DE POINTE ENDOSCOPIQUE

(30) Priority: 17.09.2020 US 202063079807 P
(43) Date of publication of application: 23.03.2022
(73) Proprietor: Gyrus ACMI, Inc. d/b/a Olympus Surgical Technologies America, Westborough, MA 01581 (US)
(72) Inventor: Shelton, Kurt G., Bedford MA 01730 (US); Bareau, Jane, Needham MA 02492 (US); Lamser, Dennis G., Marlborough MA 01752 (US); Patel, Mayur K., Framingham MA 01701 (US); McLaughlin, Anne G., Exeter NH 03833 (US); Talbot, Brian M., Southborough MA 01772 (US)
(74) Representative: Noack, Andreas

(56) References cited:
- CN-A- 111 616 773
- DE-C- 690 697
- US-A- 4 557 255

## Description

### TECHNICAL FIELD

The present application pertains generally, but not by way of limitation, to devices for endoscopic surgical procedures.

### BACKGROUND

During endoscopic urology procedures, an access sheath can be inserted into patient before inserting the endoscope. The sheath can help protect a patient's anatomy from the endoscope and from unwanted particles, such as kidney stones. The access sheath can be inserted into a patient's body until it reaches an internal surgical site, such as the ureter. The endoscope can then safely be inserted into and later withdrawn from the patient's body by passing through the interior of the access sheath. A guidewire can be used to help guide the access sheath to the internal surgical site. The guidewire can be inserted into a patient's bladder, can be passed through the ureter, and its distal portion can be inserted to enter a kidney. The access sheath can then be inserted over the guidewire to reach the kidney. US 4 557 255 A relates to a urological endoscopic device for the human body and more particularly to a ureteroscope for the operative management of the disease processes in the lower ureter under direct visual control.

### BRIEF DESCRIPTION OF THE DRAWINGS

In the drawings, which are not necessarily drawn to scale, like numerals may describe similar components in different views. Like numerals having different letter suffixes may represent different instances of similar components. The drawings illustrate generally, by way of example, but not by way of limitation, various embodiments discussed in the present document.
FIG. 1 illustrates a cross-section of an example of an endoscopic tip extender coupled to an endoscope.
FIG. 2 illustrates a cross-section of an example of an endoscopic tip extender coupled to a distal portion of an endoscope.
FIG. 3 illustrates an exploded cross-section of an example of an endoscopic tip extender and an endoscope.
FIG. 4 illustrates a cross-section of an example of the endoscopic tip extender coupled to an endoscope.
FIG. 5 illustrates an example of a method of extracting a mobile calculus from a patient using an endoscopic tip extender.

### SUMMARY/OVERVIEW

The invention is defined in claim 1. Any methods disclosed hereinafter do not form part of the scope of the invention, and are mentioned for illustrative purposes only. Endoscopes can be used to visualize or extract calculi or other target masses from various regions of a patient's body such as the urinary system, gallbladder, nasal passages, gastrointestinal tract, stomach, or tonsils. During an endoscopic procedure, an access sheath can help protect a patient's internal anatomy from the endoscope, or other surgical instruments, such as during their insertion and withdrawal. During an endoscopic urology procedure, for example, the endoscope can be passed through an access sheath previously positioned within the ureter. The endoscope can then be advanced past a distal end of the access sheath, such as to perform certain aspects of the procedure.

An endoscope can include one or more features, such as a camera, a light, and one or more working channels (e.g., a suction channel, an irrigation channel, or both). A grasping tool, such as a basket or forceps, can be inserted through the working channel of the endoscope and advanced past the distal end of the access sheath such as to capture a target particle such as a kidneys stone (a "calculus"). The captured particle can then be drawn into the working channel of the endoscope. If the particle has a greater diameter than the working channel of the endoscope, the particle can instead be drawn into the access sheath by the grasping tool. Then, the endoscope, the access sheath, and the particle captured by the grasping tool within the access sheath can all be withdrawn together concurrently.

However, if the particle is larger than the inner diameter or similar inner lateral dimension of the access sheath, the particle may break free from the grasping tool, and the endoscope, securing device, or access sheath can be damaged if a physician attempts to pull the particle through the access sheath. Additionally, modern digital endoscopes may have a larger outer diameter than fiber optic endoscopes, requiring a correspondingly larger access sheath. A larger access sheath can distend a patient's ureter, increasing patient trauma and increasing the potential of insertion problems, such bunching or folding of the distal end of the access sheath during insertion. Additionally, distention of the ureter may also lead to undesirable postprocedural complications.

Also, hydrophilic properties of an access sheath may result in the access sheath unintentionally sliding within or out of a patient during a procedure. This can create a need for a physician to reposition, or re-insert, the access sheath, which may cause trauma to the patient and lengthen the procedure. Longer procedures can inhibit or prevent proper treatment (e.g., complete ablation of calculi) and expose the patient for longer than a shorter procedure.

This disclosure can help to address these issues, among others, such as by providing an endoscopic tip extender. The endoscopic tip extender can help avoid the need for an access sheath, fora grasping device, or both, in an endoscopic procedure. The tip extender can, in place of an access sheath, extend distally from a distal portion of the endoscope such as to help protect a patient's anatomy during the insertion or removal of the endoscope. The tip extender can be detachably coupled to, or integrally formed with, the distal portion of the endoscope. For example, during an endoscopic urology procedure, the tip extender can, in place of a grasping tool, receive and encompass both a distal portion of the endoscope and one or more particles for removal, such as one or more kidney calculi, such as to both help prevent damage to a patient's anatomy and to help prevent damage to the endoscope during removal of such particles.

Additionally, the tip extender can be configured to be used with suction. For example, the tip extender can be constructed such that when a physician introduces suction through the working channel of the endoscope, to draw a particle into the tip extender, the tip extender can roll or fold inwardly. This can help to trap a particle within the tip extender. The tip extender can also help to improve the navigation of difficult or tortuous anatomical paths, as can be of shorter length, relative to an access sheath (such as may otherwise extend along the length of ureter or the calices of the kidneys). The tip extender can help provide increased flexibility, increased visibility, or both. Further, the tip extender, in contrast to an access sheath, can also be manipulated using the endoscope, such as to help push or clear obstructive particles or other matter from the path of the endoscope.

Moreover, the tip extender can also help protect a patient's anatomy during a lithotripsy procedure. In laser lithotripsy procedures, particles of larger sizes can be ablated into smaller fragments using a laser. Laser exposure and stone fragmentation can affect surrounding tissues. The tip extender can encompass particles and absorb the laser's light emissions during ablation to help protect surrounding tissue from laser exposure or fragmentation impact.

The above overview is intended to provide an overview of subject matter of the present patent application. It is not intended to provide an exclusive or exhaustive explanation of the invention. The description below is included to provide further information about the present patent application. While the following examples are discussed with a focus toward urology procedures, the endoscopic tip extender can also be used in various other endoscopic procedures.

### DETAILED DESCRIPTION

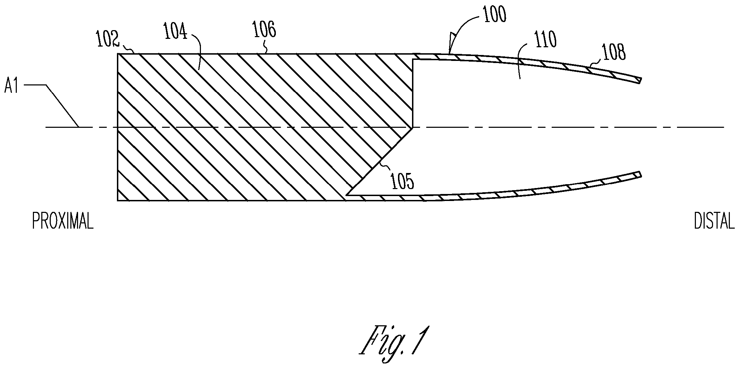

FIG. 1 illustrates a cross-section of an example of an endoscopic tip extender coupled to an endoscope. FIG. 1 includes a dashed line corresponding to a central longitudinal axis A1 defined by the endoscope, and orientation indicators "Proximal" and "Distal". As illustrated in FIG. 1, a tip extender 100 can be coupled to a distal portion of an endoscope 102. The endoscope 102 can define the longitudinal axis A1. The endoscope 102 can be any of a variety of endoscopes, such as an ureteroscope. The endoscope 102 can be a fiber optic endoscope or a digital or other electronic endoscope. The endoscope 102 can be a disposable, or a single use, endoscope. The tip extender 100 can be configured to receive a distal portion 104 of the endoscope 102, such as within a receptacle portion of the tip extender 100. For example, the tip extender 100 can radially or otherwise laterally encompass the distal portion 104. The distal portion 104 can include an objective head of the endoscope 102. The distal portion 104 can include one or more features configured to engage the tip extender 100, or vice-versa, such as further discussed below.

The distal portion 104 can include a notch 105. The notch 105 can generally be a cutout or a recess formed in the distal portion 104 of the endoscope 102. The notch 105 can extend longitudinally into the distal portion 104. The notch 105 can extend at least partially around a circumference of the distal portion 104. The tip extender 100 can include a proximal portion 106 and a distal portion 108. The proximal portion 106 can be generally cylindrical in shape. The distal portion 108 can be generally conical, frustoconical, or tapered, in shape. The tip extender 100 can taper inward toward the central longitudinal axis A1 between the proximal portion 106 and the distal portion 108. The distal portion 108 of the tip extender 100 can include a bore 110. The bore 110 can extend axially within the tip extender 100 between the proximal portion 106 and the distal portion 108, along the longitudinal axis A1. The bore 110 can be configured to receive and engage the distal portion 104. The bore 110 can be sized and shaped to create an interference fit with the distal portion 104, such as to couple the endoscope 102 to the tip extender 100. When coupled to the tip extender 100, the distal portion 104 of the endoscope 102 can extend within the proximal portion 106 of the tip extender 100, and the distal portion 108 of the tip extender 100 can extend axially and distally from the endoscope 102.

The tip extender 100 can be configured to be disposable. The tip extender 100 and the endoscope 102 can be configured to be disposable. For example, the tip extender 100 and the endoscope 102 can be integrally formed. As such, both the tip extender 100 and the endoscope 102 can be disposed of together after an endoscopic procedure. The tip extender 100 can be configured to be reusable. For example, the tip extender 100 can be configured to be reprocessed, autoclaved, or otherwise sterilized for reuse in a subsequent procedure, after being detached from the endoscope 102 following a procedure. The endoscope 102 can be configured to be reprocessed or otherwise sterilized for reuse in a future procedure.

As such, the tip extender 100, together with endoscope 102, can provide a number of benefits to a patient and to a physician. The tip extender 100 can be atraumatic such via tapering, a decreased length, or increased flexibility, or a combination thereof, relative to an access sheath, such as to allow a physician to safely perform an endoscopic procedure without using an access sheath, which can distend a patient's ureter. The tip extender 100 can help to improve both the ease of insertion into a patient and navigation through tortuous anatomical paths. The tip extender 100 can also be used to encompass a particle such as kidney stone, such as to help allow a physician to remove one or more particles without using a grasping tool, reducing the potential of damage to both a patient's anatomy and to the endoscope 102.

The tip extender 100 can be coupled to the distal portion 104 of the endoscope 102, such that the tip extender extends distally from the endoscope 102. For example, the distal portion 104 of the endoscope 102 can be inserted, into the proximal portion 106 of tip extender 100, to couple the endoscope 102 to the tip extender 100. The endoscope 102 can then be inserted into the patient. The tip extender 100 can protect a patient's anatomy from the generally blunt distal portion 104 of the endoscope 102. The tip extender 100 can trap a particle, or a portion thereof. Suction or irrigation can be introduced through a working channel of the endoscope 102. For example, the suction can draw the particle into the tip extender 100, and can also cause a distal end of the tip extender 100 to fold inwardly, helping to retain the particle therein. The endoscope 102 can then be removed from the patient. The tip extender 100 can protect the patient's anatomy from any rough or sharp surfaces of the particle during removal. After the procedure, the tip extender 100 can be decoupled from the endoscope 102. For example, the tip extender 100 can be pulled in a distal direction, while the endoscope 102 remains stationary, to remove the distal portion 104 of the endoscope 102 from the tip extender 100. The tip extender 100 can be subsequently disposed of, or can be reprocessed or otherwise sterilized for reuse, along with the endoscope 102.

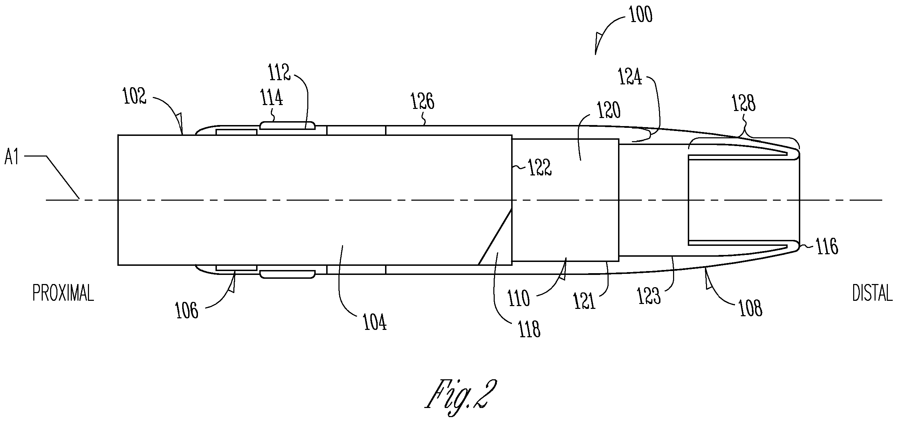

FIG. 2 illustrates a cross-section of an example of the endoscopic tip extender 100 and a distal portion 104 of the endoscope 102. Also shown in FIG. 2 is a central longitudinal axis A1, and orientation indicators Proximal and Distal. As illustrated in FIG. 2, the tip extender 100 can include a lock groove 112. The lock groove 112 can be a circumferentially or otherwise peripherally formed groove in the proximal potion 106 of the tip extender 100. The lock groove 112 can be formed transversely to the longitudinal axis A1. The lock groove 112 can be laterally located at any of various locations along the proximal portion 106 of the tip extender 100. The lock groove 112 can be configured to accept a lock ring 114.

The lock ring 114 can generally be a semi-circular lock ring, or another type of lock collar. The lock ring 114 can be positioned over the proximal portion 106 of the tip extender 100, within the lock groove 112. When positioned within the lock groove 112, the lock ring 114 can face inwardly to engage and squeeze the proximal portion 106 of the tip extender 100. The lock groove 112 can laterally retain and positon the lock ring 114 on the proximal portion 106. The lock ring 114 can be made from by swaging, molding, or machining from various materials, including, but not limited to stainless steel. As such, the lock ring 114, together with the lock groove 112, can help to securely couple the tip extender 100 to the endoscope 102.

The distal portion 108 of the tip extender 100 can include a distal end 116. The tip extender 100 can taper between the distal portion 108 and the distal end 116. For example, the proximal portion 106 can have an outer diameter of about 3.0-3.5 mm, 3.5- 3.9 mm, or 3.2-4.0 mm. The distal portion 108 portion can taper down, from the diameter of the proximal portion 106, to an outer diameter of about 2.0 mm-2.5 mm, 2.5 mm-2.9 mm, or 2.0mm-3.0 mm at or near the distal end 116. The distal portion 108 can also taper down over various longitudinal distances. For example, the distal portion 108 can taper down over a length of 0.5-1 mm, 1-5 mm, 5-8 mm, or 0.5-8 mm. The distal end 116 can be angled orthogonally, relative to the longitudinal axis A1 and to the bore 110 of the tip extender 100. The distal end 116 of the distal portion 108 can be angled obliquely, relative to the longitudinal axis A1 and to the bore 110 of the tip extender 100. For example, the distal end 116 can be obliquely angled at, but not limited to, 110, 120, 130, 140, 150 or 160 degrees relative to the longitudinal axis A1.

The bore 110 can include a first portion 118 and a second portion 120. The first portion 118 and the second portion 120 of the bore 110 can generally define opposite proximal and distal portions of the bore 110, respectively. The first portion 118 can extend axially within the proximal portion 106. The first portion 118 can have a generally cylindrical shape. The first portion 118 can be configured to engage the distal portion 104 of the endoscope 102. The first portion 118 can be sized and shaped to create an interference fit along a length of the distal portion 104 of the endoscope 102. The first portion 118 can taper distally toward the second portion 120. The first portion 118 can thereby limit distal translation of the distal portion 104 of the endoscope 102 within the bore 110, to position the distal portion 104 within the proximal portion 106 of the tip extender 100.

The first portion 118 can include a ridge 122. The ridge 122 can be a protrusion or ridge extending radially into the first portion 118 of the bore 110. The ridge 122 can extend at least partially around a circumference of the first portion 118. The ridge 122 can also separate the first portion 118 from the second portion 120 of the bore 110. The first portion 118 can thereby limit distal translation of the distal portion 104 of the endoscope 102 within the bore 110, to position the distal portion 104 within the proximal portion 106 of the tip extender 100. For example, the endoscope 102 can be inserted into the bore 110 until the distal portion 104 contacts the ridge 122, preventing further distal translation of the endoscope 102 within the tip extender 100.

The second portion 120 of the bore 110 can extend axially within distal portion 108. The second portion 120 can have a generally cylindrical shape. The second portion 120 can be configured to accept, retain, or otherwise encompass a particle, or retain a portion thereof. The second portion 120 of the bore 110 can have a reduced diameter relative to the first portion 118. The second portion 120 can be tapered toward the distal end 116 of the distal portion 108. The second portion 118 of the bore 110 can include also be stepped. For example, the second portion 120 can include a first diameter 121 and a second diameter 123. The first diameter 121 can extend distally from a distal end of the first portion 118, to the second diameter 123. The second diameter can 123 can extend distally from a distal end of the first diameter 121 to the distal end 116. The second diameter 123 can have a reduced diameter, relative to the first diameter 121.

The tip extender 100 can include a wall thickness 124. The wall thickness 124 can be defined as the vertical distance between an outer surface 126 of the tip extender 100 and the bore 110. For example, the wall thickness 124 can be about 0.10-0.15 mm, 0.15-0.20mm, or 0.10-0.20 mm. The wall thickness 124 can taper, or decrease, between the proximal portion 106 and at the distal portion 108. For example, the proximal portion 106 can have a wall thickness of about 0.20 mm and the distal portion can have a wall thickness of about 0.15 mm. A tapered wall thickness 124 can provide the benefit of allowing the tip extender 100 to securely engage the distal portion 104 of the endoscope 102, while simultaneously allowing the distal portion 108 to have an increased flexibly relative to the proximal portion 106.

The tip extender 100 can also form a fold-back 128. The fold-back 128 can be defined as an orientation or a position of the distal end 116. For example, the distal end 116 can roll or flex proximally and inwardly to form the fold-back 128. The fold-back 128 can help to trap or retain a particle, such as a renal calculus (kidney stone), within the second portion 120 of the bore 110. For example, a physician can introduce suction generated by an external device, though the endoscope 102. The vacuum generated by the external device can draw the particle into the second portion 120 of the bore 110. The vacuum can cause the distal end 116 to roll proximally and inwardly to form the fold-back 128, to trap the particle within the tip extender 100.

The tip extender 100 can be made by molding, swaging, extruding, or machining from any of a variety of materials, including but not limited to, rubber, plastic, silicone, or other polymers. The tip extender 100 can be made from a substantially softer or more flexible material, relative to the distal portion 104 of the endoscope 102. For example, the tip extender 100 can be made from a material having a durometer of about, but not limited to, 50-55a, 56a-60a, 61-69a, or 70a-80a. The tip extender 100 can include materials of variable durometer hardness. For example, the proximal portion 106 can be made from a softer durometer and the distal portion 108 can be made from a firmer durometer. A softer durometer can increase the flexibility of the tip extender 100, for example, to improve the ease of insertion through tortuous anatomical pathways and improve the ability of the tip extender 100 to flex, to accept a greater variety of particles. A firmer durometer can improve the ease of manipulation when clearing obstructions or other matter from the path of the endoscope 102, and can help to improve engagement between the tip extender 100 and the endoscope 102, to help to prevent the tip extender 100 from unintentionally releasing the endoscope 102.

The tip extender 100 can also be made from a heat moldable material, such as a thermoset or thermoplastic elastomer, such as to allow a physician to manually shape the tip extender 100. For example, the distal end 116 of the tip extender 100 can be rolled inwardly, by hand, and then heat-set form the fold-back 128 without the use of suction. The tip extender 100 could also be heated and manipulated into a curved shape, to aid in trapping a particle against, for example, a wall of patient's ureter or a wall of a calyx in a kidney.

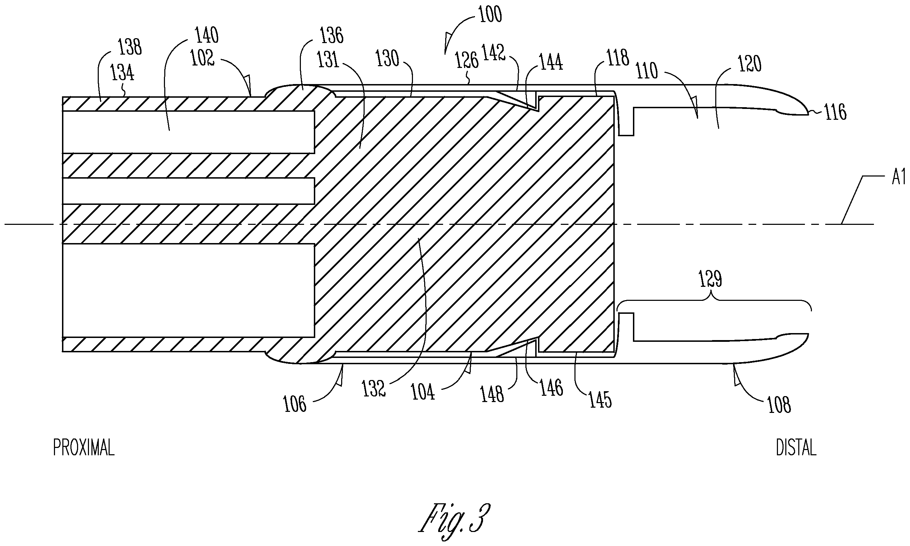

FIG. 3 illustrates an exploded cross-section of an example of the endoscopic tip extender 100 and the endoscope 102. Also shown in FIG. 3 is a longitudinal axis A1, and orientation indicators Proximal and Distal. As illustrated in FIG. 3, the tip extender 100 can define a distal extension 129. The distal extension 129 can be defined as the axial distance the tip extender 100 extends from the distal portion 104 when coupled to the endoscope 102.

For example, the distal extension 129 can be 0.5-1 mm, 1-5 mm, 5-8 mm, or 0.5-8 mm. The tip extender 100 can be configured to for a reduced distal extension 129, such as 1-5mm, to help to increase flexibility in reaching difficult to access locations, such as the calices of a patient's kidney, and field of view of for a camera positioned on the distal portion 104 of the endoscope 102, thereby helping to improve intra-procedural visibility for a physician or user operating the endoscope 102. The tip extender 100 can be configured to define a greater distal extension 129, such as about 5-8mm to accommodate or trap larger particles for within the tip extender 100. Additionally, the tip extender 100 can also be made from a firmer material when configured to define a greater distal extension 129, in order to increase the structural integrity of the tip extender 100, to help in manipulating resistant particles or other matter, such as calculi or plasma, without folding inwardly or otherwise bending or deflecting.

The endoscope 102 can include an objective head 130, an imaging device 131, a working channel 132, a string 134, an adhesive 136, shrink tubing 138, and a deflection tube 140. The objective head 130 and the imaging device 131, along with the working channel 132, the string 134, the adhesive 136, the shrink tubing 138, and the deflection tube 140, can be standard components of an endoscope. The distal portion 104 of the endoscope 102 can comprise, or include, the objective head 130. The objective head 130 can be made from a variety of rigid or substantially rigid materials including, but not limited to, plastic, ceramic, or stainless steel. The objective head 130 can be made from a substantially harder, firmer, or otherwise less pliable material, relative to the tip extender 100. The objective head 130 can include the imaging device 131. The imaging device 131 can be located on a distal end of the objective head 130, or distal portion 104, and can include at least a camera and one or more lights. The imaging device 131 can also include electronics components or wiring extending axially within the endoscope 102, in a proximal direction from the objective head 130 to a proximal end of the endoscope 102.

The working channel 132 can be a bore extending along the longitudinal axis A1 within the endoscope 102. The working channel 132 can be configured accept a surgical instrument 134. The surgical instrument 134 can be a variety of surgical tools, such as an endoscopic forceps, a grasping tool, or a retaining basket. The working channel 132 can also be configured to withstand suction introduced by an external device or carry irrigation fluid axially through the endoscope 102. The string 134 can couple the objective head 130 to external user controls, to allow a physician or user to manipulate the objective head 130 from a location external to a patient. The string 134 can be made from, but not limited to, cloth, twine, metals or other materials. The string 134 can be coupled to the objective head with an adhesive 136.

The adhesive 136 can be an adhesive applied to the objective head 130, the string 134, and to the shrink tubing 138. The adhesive 136 can form a protrusion extending radially outward and circumferentially around the objective head 130 and the shrink tubing 138. The shrink tubing 138 can encompass the string 134 to protect the string 134. The shrink tubing 138 can also form an outer surface of the endoscope 102, proximal to the objective head 130. The deflection tube 140 can generally form a core of the endoscope 102, proximal to the objective head 130. As such, the imaging device 131 and the working channel 132 can be formed in, and extend through, the deflection tube 140.

The tip extender can include at least a first protrusion 142. The first protrusion 142 can extend radially outwardly from the proximal portion 106 of the tip extender 100. The first protrusion 142 can extend at least partially around a circumference of the proximal portion 106 of the tip extender 100. The first protrusion 142 can have a generally triangular or wedge-shaped, but can also form other three-dimensional shapes such as ellipses, rectangles or cubes. The first protrusion 142 can be shaped to correspondingly engage a first recess 144. The endoscope 102 can define a first recess 144. The first recess 144 can be formed in an outer surface 145 of the distal portion 104 or the objective head 130, of the endoscope 102. The distal portion 104 of the endoscope 102 can define a second recess 146 in the outer surface 145. The second recess 146 recess be axially spaced laterally, or longitudinally, apart from the first recess 144 along the longitudinal axis A1. As such, the second recess 146 can allow the distal extension 129 of the tip extender 100 to be selectively adjustable, based on whether a user chooses to engage the first 144 and second 146 recesses with the first protrusion 142.

The tip extender 100 can also include a second protrusion 148. When the tip extender 100 includes a second protrusion 148, the second recess 146 (and the second protrusion 148) can be circumferentially offset in the outer surface 145 of the distal portion 104, relative to the first protrusion 142 and the first recess 144. For example, the second recess 146 and the second protrusion 148 can be located at 60-90, 100-180, or 190-270 degrees, relative to the first protrusion 142 and to the first recess 144.

In some examples, the tip extender 100 can further include additional protrusions and corresponding recesses. For example, the tip extender 100 can include a third and a fourth protrusion, and the distal portion 104 of the endoscope 102 can define a third and a fourth recess. The third protrusion can be axially aligned with, but laterally offset from, the first protrusion 142 and the first recess 144; and the fourth protrusion can be axially aligned with, but laterally offset from, the second protrusion 148 and the second recess 146. As such, this can allow the distal extension 129 of the tip extender 100 to be selectively adjustable while also strengthening the coupling between the tip extender 100 and the endoscope 102.

In the operation of some examples, a user can couple the tip extender 100 to the endoscope 102 by translating the objective head 130 distally within the bore 110 of the tip extender 100, until the first protrusion 142 and the second protrusion 148 engage the first recess 144 and the second recess 146, respectively. The first protrusion 142 and the second protrusion 148 can slide along the outer surface 145 of the objective head, causing the tip extender 100 to flex outwardly. When the first protrusion 142 and the second protrusion 148 reach the first recess 144 and the second recess 146, respectively, the tip extender 100 can flex inwardly as the first protrusion 142 enters the first recess 144 and the second protrusion 148 enters the second recess 146, creating a snap fit between the tip extender 100 and the objective head 130 of the endoscope 102.

The tip extender 100 can be transparent, translucent, or otherwise semitransparent to further increase visibility of internal anatomy over an access sheath. For example, the tip extender 100 can allow visible light, for example, light about 380-700 nanometers in wavelength, to pass through the outer surface 126 of the tip extender 100, such as light provided by the objective head 130 or the imaging device 131. In some examples, a transparent tip extender 100 can allow a user to view internal anatomy distinctly through the outer surface 126 of the tip extender 100, for example, using the imaging device 131. The tip extender 100 can be configured to absorb laser light during laser ablation of a calculus. The tip extender 100 can absorb, for example, light emitted from a 2000 nanometer wavelength infrared laser. The tip extender 100 can help to keep obstructions away from the distal portion 104 of the endoscope 102, which can often block imaging from the imaging device 131 and clog the working channel 132 of the endoscope 102.

The tip extender 100 can provide a number of benefits to both a physician and a patient during an endoscopic procedure. The tip extender 100 can be atraumatic such via tapering, a decreased length, or increased flexibility, or a combination thereof, relative to an access sheath, and can receive and encompass both the objective head 130 of the endoscope 102, and also particles to be removed, such as kidney stones, to eliminate the need for both an access sheath, and a grasping tool, during a urology procedure. The short length of the tip extender 100, relative to a traditional access sheath, can also improve a user's intra-procedural view of a patient's internal anatomy at an anatomical site. Anatomical features, such as bends in the ureter, can be viewed directly through the distal end 116 of the tip extender 100, instead of through a wall of an access sheath, thereby improving the ease of navigation through tortuous anatomical paths.

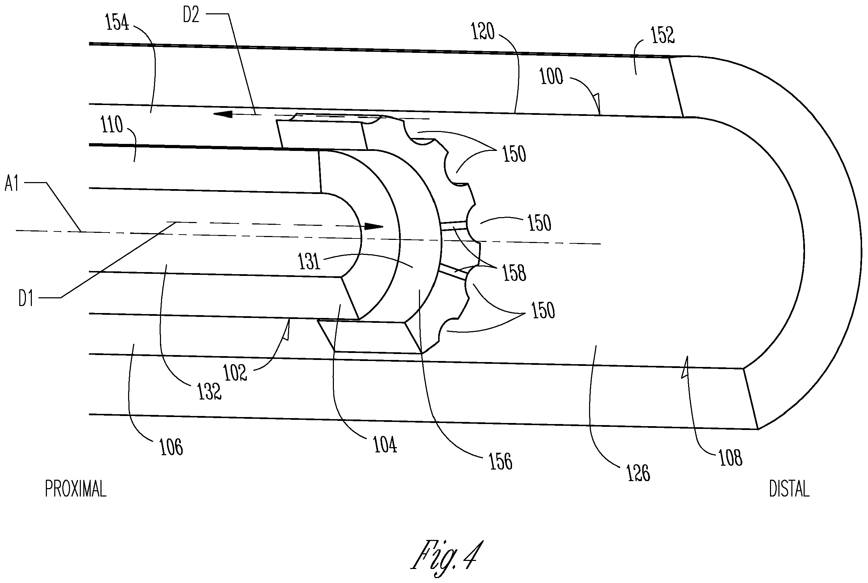

FIG. 4 illustrates a cross-section of an endoscope 102 and an endoscopic tip extender 100. Also shown in FIG. 4 is a longitudinal axis A1, and orientation indicators Proximal and Distal. As illustrated in FIG. 4, the outer surface 126 of the tip extender 100 can define one or more drainage or suction grooves 150. The grooves 150 can be concave channels extending parallel to the longitudinal axis A1 between the proximal portion 106 and the distal portion 108, of the tip extender 100.

In the operation of some examples, the endoscope 102, including the working channel 132, can be configured to allow a user to actively introduce irrigation fluid into a patient's anatomy. Directional indicator D1 indicates the direction of irrigation fluid flow through the working channel 132. The irrigation fluid can be, for example, a saline solution. In order to avoid over-pressurizing the anatomical site, such as a ureter, the irrigation fluid must be have access to an outlet. The grooves 150 can help to allow irrigation fluid to drain in a proximal direction around the tip extender 100, between the outer surface 126 and an anatomical wall 152 of a patient. Directional indicator D2 indicates the direction of irrigation fluid drainage, through the grooves 150. The anatomical wall 152, for example, can be a wall of a ureter or a calyx of a kidney. The grooves 150 can used with both a passive drainage system (gravity); or active drainage system (suction).

If an active drainage system is to be used, the *in situ* pressure can be monitored, such as to ensure the pressure is within specified limits. Active introduction of irrigation fluid, or active suction for irrigation drainage, can result in respective positive or negative pressure changes in an anatomical site. Negative or positive pressure changes, if not properly controlled, may affect internal organs or other anatomy. The internal pressure can be monitored by various systems or methods. For example, one or more pressure sensors can be used in conjunction with a flow sensor and an external control module to monitor *in situ* pressure.

The *in situ* pressure can be monitored at various locations at or near the tip extender 100. For example, pressure can be monitored with a first pressure sensor, such as a membrane sensor, at a first site 154. The first site 154 can be a point located along the outer surface 126 of the proximal portion 106, of the tip extender 100. Pressure can also be monitored at second site 156. The second point 156 can be a point located on the distal portion 104 of the endoscope 102, within the bore 110 of the tip extender 100. Both the first point 154 and the second point 156 can also be monitored concurrently, in a delayed, or in a time-interleaved fashion, to observe a pressure differential between the first site 154 and the second site 156. Monitoring a differential pressure can help increase the accuracy of any pressure readings obtained from an anatomical site.

A flow sensor can be used to sense an irrigation fluid flow rate through the working channel 132 of the endoscope 102. An external control module can be configured to detect the flow rate, which can indicate a presence or an absence of a clog in the working channel 132. In response to the sensed fluid flow rate through the working channel 132, the external control module can control one or more of an irrigation source or a suction source to provide suitable irrigation fluid flow, or suction pressure, through the working channel 132. For example, the external control module can apply suction to unclog the working channel 132. The external control module 132 can also automatically adjust one or more of the irrigation fluid flow rate, or a suction flow rate, through the working channel 132 to maintain the pressure of the anatomical site at substantially a desired pressure level (e.g., a predetermined or a user-specified pressure level).

The use of irrigation can provide numerous benefits to both an operating physician and to a patient. Irrigation can help to dislodge and facilitate the removal of particles such as calculi, tissue debris, stone fragments, or other unwanted matter through the working channel 132. Irrigation fluid introduction can also help to maintain clear visibility of the anatomical site through the imaging 131. Additionally, irrigation fluid flow can have a cooling effect on an endoscopic tissue removal device, and can also help to dissipate heat generated during laser ablation of calculi or other particles.

Additionally, the grooves 150 of the tip extender 100 can include one or more passages 158. The passages 158 can be bores extending radially inwardly between the grooves 150 and the bore 110. The passages 158 can be generally cylindrical in shape, but can also be other three-dimensional shapes such as rectangular, hexagonal, or trapezoidal prisms. When active drainage is used, the grooves 150, together with the passages 158, can help to prevent a reduction in suction through the working channel 132 of the tip extender 100. For example, if a captured particle or other matter substantially or completely occludes the second portion 120 of the bore 110; suction applied through the working channel 132 can alternatively reach the grooves 150 located along the outer surface 126 of the tip extender 100 via the passages 158.

The grooves 150 can provide numerous benefits to both an operating physician and to a patient. The tip extender 100 can provide a secondary route for irrigation fluid drainage, helping increase the rate of irrigation fluid introduction without increasing the pressure on an anatomical site. For example, when an endoscope is used with an access sheath, bi-directional irrigation fluid flow is limited by combined introduction and drainage through one or more working channels 132 of the endoscope 102. A slow or significantly limited irrigation flow rate can be less efficient in flushing out unwanted matter from the anatomical site, which can result in reduced visibility and can increase the likelihood of a clog in the working channel 132. Reduced irrigation volume and flow rate may also limit cooling effects on surgical tools and increase the chance of undesirable heat accumulation at the anatomical site.

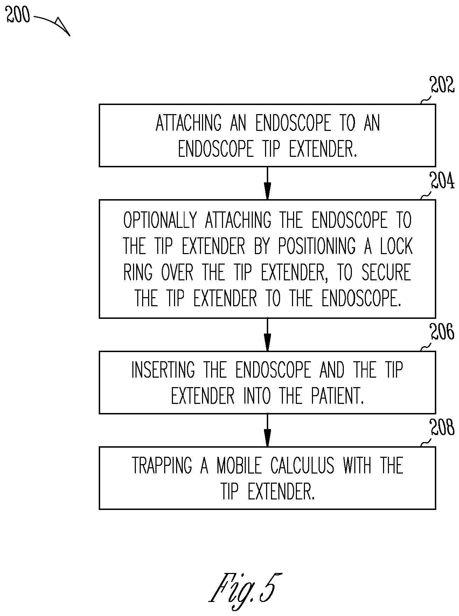

FIG. 5 illustrates an example of a method of extracting a mobile calculus from a patient. In this example, the method 200 includes operations such as attaching an endoscope to an endoscope tip extender at 202, optionally positioning a lock ring over the tip extender at 204, inserting the endoscope and the tip extender into a patient at 206, and trapping a mobile calculus at 208. In one or more examples, the method 200 can begin at step 202 with a user attaching an endoscope to an endoscope tip extender. For example, as shown in FIGS. 1-2, a user can insert the distal portion 104 of the endoscope 102 into the bore 110, of the tip extender 100.

In one or more examples, an optional second step 204 can be positioning a lock ring over the tip extender, to further secure the tip extender to the endoscope. For example, a user can position the lock ring 114 over the tip extender 100; within the lock groove 112. In one or more examples, a third step 206 can be inserting the endoscope and the tip extender into the patient. For example, a user can insert the tip extender 100 and the endoscope 102 through the bladder and into the ureter of a patient. In one or more examples, a fourth step can be trapping a mobile calculus with the tip extender. For example, a user can manipulate the distal portion 104 of the endoscope 102, and also apply suction through the working channel 132, to draw the mobile calculus into the tip extender 100 and trap the mobile calculus for safe removal. In some examples, the distal end 116 of the tip extender 100 can roll inwardly, in response to suction, to form the fold-back 128, to further retain the mobile calculus within the tip extender 100. Such a method can be repeated, as desired.

The steps or operations of the method 200 are illustrated in a particular order for convenience and clarity. The discussed operations can be performed in parallel or in a different sequence without materially impacting other operations. The method 200 as discussed includes operations that can be performed by multiple different actors, devices, and/or systems. It is understood that subsets of the operations discussed in the method 200 can be attributable to a single actor device, or system, and could be considered a separate standalone process or method.

The tip extender 100 of the present disclosure can help avoid the use of access sheath during an endoscopic procedure. Eliminating the use of an access sheath can help prevent undesirable dilation of the ureter, and access sheath movement or slippage within a patient, while protecting a patient's anatomy from a distal portion 104 of an endoscope during insertion and removal. The tip extender 100 can be easily manipulated by a user, and can used with or without a suction device to trap a calculus, without the use of a grasping tool. The tip extender 100 can help increase visibility of an anatomical site, relative to an access sheath, and can encompass a calculus during laser ablation to protect surrounding tissue from laser and heat exposure. Finally, the tip extender 100 can increase the flow rate of irrigation introduction and drainage from a patient's anatomy during an endoscopic procedure such as by providing a secondary drainage path.

The above detailed description includes references to the accompanying drawings, which form a part of the detailed description. The drawings show, by way of illustration, specific embodiments in which the invention can be practiced. These embodiments are also referred to herein as "examples." Such examples can include elements in addition to those shown or described. However, the present inventors also contemplate examples in which only those elements shown or described are provided. Moreover, the present inventors also contemplate examples using any combination or permutation of those elements shown or described (or one or more aspects thereof), either with respect to a particular example (or one or more aspects thereof), or with respect to other examples (or one or more aspects thereof) shown or described herein.

In this document, the terms "a" or "an" are used, as is common in patent documents, to include one or more than one, independent of any other instances or usages of "at least one" or "one or more." In this document, the term "or" is used to refer to a nonexclusive or, such that "A or B" includes "A but not B," "B but not A," and "A and B," unless otherwise indicated. In this document, the terms "including" and "in which" are used as the plain-English equivalents of the respective terms "comprising" and "wherein." Also, in the following claims, the terms "including" and "comprising" are open-ended, that is, a system, device, article, composition, formulation, or process that includes elements in addition to those listed after such a term in a claim are still deemed to fall within the scope of that claim. Moreover, in the following claims, the terms "first," "second," and "third," etc. are used merely as labels, and are not intended to impose numerical requirements on their objects.

The above description is intended to be illustrative, and not restrictive. For example, the above-described examples (or one or more aspects thereof) may be used in combination with each other. Other embodiments can be used, such as by one of ordinary skill in the art upon reviewing the above description. The Abstract is provided to allow the reader to quickly ascertain the nature of the technical disclosure. It is submitted with the understanding that it will not be used to interpret or limit the scope or meaning of the claims. Also, in the above Detailed Description, various features may be grouped together to streamline the disclosure.

This should not be interpreted as intending that an unclaimed disclosed feature is essential to any claim. Rather, inventive subject matter may lie in less than all features of a particular disclosed embodiment. Thus, the following claims are hereby incorporated into the Detailed Description as examples or embodiments, with each claim standing on its own as a separate embodiment, and it is contemplated that such embodiments can be combined with each other in various combinations or permutations. The scope of the invention should be determined with reference to the appended claims, along with the full scope to which such claims are entitled.

## Claims

1. An atraumatic endoscopic medical apparatus, comprising:
an endoscope;
an endoscope tip extender (100), configured to be attached to and extend longitudinally axially from a distal portion (104) of said endoscope (102), the tip extender defining a bore (110) extending longitudinally axially within the tip extender, the tip extender defining a tapered arrangement and configured to trap a mobile calculus within the bore of the tip extender, **characterised by** said tip extender including a material that is at least one of softer or more flexible than a material of the distal portion of the endoscope.

2. The apparatus of claim 1, further comprising the endoscope, wherein the tip extender is configured to be releasably coupled to the endoscope.

3. The apparatus of claim 2, wherein the bore of the tip extender is configured to accept the distal portion of the endoscope, the bore configured to engage an outer surface (145) of the distal portion of the endoscope to releasably couple the tip extender to the endoscope.

4. The apparatus of claim 2 or 3, further comprising a lock groove (112) circumferentially formed in a proximal portion (106) of the tip extender, the lock groove configured to receive a lock ring (114).

5. The apparatus of claim 4, wherein the tip extender is releasably coupled to the endoscope with a lock ring, the lock ring engaging the tip extender within the lock groove, to couple the proximal portion of the tip extender to the distal portion of the endoscope.

6. The apparatus of claim 2, wherein the tip extender includes a protrusion (142, 148) extending radially outward from the proximal portion of the tip extender, the protrusion configured to correspondingly engage a recess (144, 146) in the distal portion of the endoscope to releasably couple the tip extender to the endoscope.

7. The apparatus of claim 6, wherein the endoscope further comprises a first recess and a second recess in the distal portion of the endoscope, the first recess and the second recess spaced longitudinally apart, such that the tip extender can be adjustably coupled to the endoscope by engaging either the first recess or the second recess.

8. The apparatus of claim 1, wherein the tip extender is integrally formed with the distal portion of the endoscope, the endoscope and the tip extender together configured to be disposable.

9. The apparatus of any of claims 1 to 8, wherein a distal portion (108) of the tip extender is angled obliquely relative to the bore of the tip extender.

10. The apparatus of any of claims 1 to 9, wherein the bore of the tip extender is tapered down from a proximal portion of the bore to a distal portion of the bore.

11. The apparatus of any of claims 1 to 10, wherein the bore of the tip extender includes a first portion (118) and a second portion (120), the first portion having a greater diameter than the second portion, the first portion configured to receive the distal portion of the endoscope at a proximal portion of the tip extender, and the second portion configured to limit distal translation of the endoscope within the tip extender.

12. The apparatus of claim 11, wherein the second portion of the bore is tapered, and is configured to flex inwardly to accept and retain a mobile calculus.

13. The apparatus of any of claims 1 to 12, wherein the tip extender includes one or more grooves (150) extending longitudinally axially along an outer surface (126) of the tip extender.

14. The apparatus of claim 13, wherein the tip extender includes one or more passages (158) extending radially inwardly between the one or more grooves and the bore.

15. The apparatus of any of claims 1 to 14, wherein at least a portion of the tip extender is transparent or translucent.

## Patentansprüche

1. Eine atraumatische endoskopische Medizinische Vorrichtung, umfassend:
ein Endoskop;
eine Endoskopspitzenverlängerung (100), die dazu eingerichtet ist, an einem distalen Abschnitt (104) des Endoskops (102) befestigt zu werden und sich in Längsrichtung axial von diesem zu erstrecken, wobei die Spitzenverlängerung eine Bohrung (110) definiert, die sich in Längsrichtung axial innerhalb der Spitzenverlängerung erstreckt, wobei die Spitzenverlängerung eine sich verjüngende Anordnung definiert und dazu eingerichtet ist, einen beweglichen Stein innerhalb der Bohrung der Spitzenverlängerung einzufangen, **dadurch gekennzeichnet, dass** die Spitzenverlängerung ein Material enthält, das zumindest entweder weicher oder flexibler ist als ein Material des distalen Abschnitts des Endoskops.

2. Die Vorrichtung nach Anspruch 1, ferner umfassend das Endoskop, wobei die Spitzenverlängerung dazu eingerichtet ist, lösbar mit dem Endoskop verbunden zu werden.

3. Die Vorrichtung nach Anspruch 2, wobei die Bohrung der Spitzenverlängerung dazu eingerichtet ist, den distalen Abschnitt des Endoskops aufzunehmen, wobei die Bohrung dazu eingerichtet ist, mit einer Außenfläche (145) des distalen Abschnitts des Endoskops in Eingriff zu kommen, um die Spitzenverlängerung lösbar mit dem Endoskop zu verbinden.

4. Die Vorrichtung nach Anspruch 2 oder 3, ferner umfassend eine Verriegelungsnut (112), die in einem proximalen Abschnitt (106) der Spitzenverlängerung in Umfangsrichtung ausgebildet ist, wobei die Verriegelungsnut so eingerichtet ist, dass sie einen Verriegelungsring (114) aufnimmt.

5. Die Vorrichtung nach Anspruch 4, wobei die Spitzenverlängerung mit einem Verriegelungsring lösbar an das Endoskop gekoppelt ist, wobei der Verriegelungsring mit der Spitzenverlängerung innerhalb der Verriegelungsnut in Eingriff steht, um den proximalen Abschnitt der Spitzenverlängerung mit dem distalen Abschnitt des Endoskops zu koppeln.

6. Die Vorrichtung nach Anspruch 2, wobei die Spitzenverlängerung einen Vorsprung (142, 148) aufweist, der sich von dem proximalen Abschnitt der Spitzenverlängerung radial nach außen erstreckt, wobei der Vorsprung so eingerichtet ist, dass er entsprechend in eine Aussparung (144, 146) in dem distalen Abschnitt des Endoskops eingreift, um die Spitzenverlängerung lösbar mit dem Endoskop zu verbinden.

7. Die Vorrichtung nach Anspruch 6, wobei das Endoskop ferner eine erste Aussparung und eine zweite Aussparung im distalen Abschnitt des Endoskops umfasst, wobei die erste Aussparung und die zweite Aussparung in Längsrichtung voneinander beabstandet sind, so dass die Spitzenverlängerung einstellbar mit dem Endoskop gekoppelt werden kann, indem sie entweder in die erste Aussparung oder die zweite Aussparung eingreift.

8. Die Vorrichtung nach Anspruch 1, wobei die Spitzenverlängerung einstückig mit dem distalen Teil des Endoskops ausgebildet ist, wobei das Endoskop und die Spitzenverlängerung zusammen so eingerichtet sind, dass sie wegwerfbar sind.

9. Die Vorrichtung nach einem der Ansprüche 1 bis 8, wobei ein distaler Abschnitt (108) der Spitzenverlängerung schräg zur Bohrung der Spitzenverlängerung abgewinkelt ist.

10. Die Vorrichtung nach einem der Ansprüche 1 bis 9, wobei sich die Bohrung der Spitzenverlängerung von einem proximalen Abschnitt der Bohrung zu einem distalen Abschnitt der Bohrung hin verjüngt.

11. Die Vorrichtung nach einem der Ansprüche 1 bis 10, wobei die Bohrung der Spitzenverlängerung einen ersten Abschnitt (118) und einen zweiten Abschnitt (120) aufweist, wobei der erste Abschnitt einen größeren Durchmesser als der zweite Abschnitt hat, wobei der erste Abschnitt dazu eingerichtet ist, den distalen Abschnitt des Endoskops an einem proximalen Abschnitt der Spitzenverlängerung aufzunehmen, und wobei der zweite Abschnitt dazu eingerichtet ist, die distale Translation des Endoskops innerhalb der Spitzenverlängerung zu begrenzen.

12. Die Vorrichtung nach Anspruch 11, wobei der zweite Abschnitt der Bohrung konisch ist und dazu eingerichtet ist, sich nach innen zu biegen, um einen beweglichen Stein aufzunehmen und festzuhalten.

13. Die Vorrichtung nach einem der Ansprüche 1 bis 12, wobei die Spitzenverlängerung eine oder mehrere Rillen (150) aufweist, die sich in Längsrichtung axial entlang einer Außenfläche (126) der Spitzenverlängerung erstrecken.

14. Die Vorrichtung nach Anspruch 13, wobei die Spitzenverlängerung einen oder mehrere Durchgänge (158) aufweist, die sich radial nach innen zwischen der einen oder den mehreren Nuten und der Bohrung erstrecken.

15. Die Vorrichtung nach einem der Ansprüche 1 bis 14, wobei mindestens ein Teil der Spitzenverlängerung transparent oder durchscheinend ist.

## Revendications

1. Appareil médical endoscopique atraumatique, comprenant :
un endoscope ;
un prolongateur de pointe d'endoscope (100), configuré pour être fixé à une partie distale (104) de l'endoscope (102) et s'étendre longitudinalement axialement à partir de celle-ci, le prolongateur de pointe définissant un alésage (110) s'étendant longitudinalement axialement à l'intérieur du prolongateur de pointe, le prolongateur de pointe définissant un arrangement conique et configuré pour piéger un calcul mobile à l'intérieur de l'alésage du prolongateur de pointe, **caractérisé par le fait que** ledit prolongateur de pointe comprend un matériau qui est au moins l'un des suivants : plus souple ou plus flexible qu'un matériau de la partie distale de l'endoscope.

2. L'appareil de la revendication 1, comprenant en outre l'endoscope, dans lequel le prolongateur de pointe est configuré pour être couplé de manière libérable à l'endoscope.

3. L'appareil de la revendication 2, dans lequel l'alésage du prolongateur de pointe est configuré pour accepter la partie distale de l'endoscope, l'alésage étant configuré pour s'engager dans une surface extérieure (145) de la partie distale de l'endoscope pour coupler de manière libérable le prolongateur de pointe à l'endoscope.

4. L'appareil de la revendication 2 ou 3, comprenant en outre une rainure de verrouillage (112) formée circonférentiellement dans une partie proximale (106) du prolongateur de pointe, la rainure de verrouillage configurée pour recevoir une bague de verrouillage (114).

5. L'appareil de la revendication 4, dans lequel le prolongateur de pointe est couplé de manière amovible à l'endoscope avec un anneau de verrouillage, l'anneau de verrouillage s'engageant dans le prolongateur de pointe à l'intérieur de la rainure de verrouillage, pour coupler la partie proximale du prolongateur de pointe à la partie distale de l'endoscope.

6. L'appareil de la revendication 2, dans lequel le prolongateur de pointe comprend une protubérance (142, 148) s'étendant radialement vers l'extérieur à partir de la partie proximale du prolongateur de pointe, la protubérance configurée pour engager de manière correspondante un renfoncement (144, 146) dans la partie distale de l'endoscope pour coupler de manière libérable le prolongateur de pointe à l'endoscope.

7. L'appareil de la revendication 6, dans lequel l'endoscope comprend en outre un premier renfoncement et un second renfoncement dans la partie distale de l'endoscope, le premier renfoncement et le second renfoncement étant espacés longitudinalement, de sorte que le prolongateur de pointe peut être couplé de manière ajustable à l'endoscope en engageant soit le premier renfoncement, soit le second renfoncement.

8. L'appareil de la revendication 1, dans lequel le prolongateur de pointe est formé intégralement avec la partie distale de l'endoscope, l'endoscope et le prolongateur de pointe étant configurés ensemble pour être jetables.

9. L'appareil de l'une des revendications 1 à 8, dans lequel une partie distale (108) du prolongateur de pointe est inclinée obliquement par rapport à l'alésage du prolongateur de pointe.

10. L'appareil de l'une des revendications 1 à 9, dans lequel l'alésage du prolongateur de pointe est conique depuis une partie proximale de l'alésage jusqu'à une partie distale de l'alésage.

11. L'appareil de l'une des revendications 1 à 10, dans lequel l'alésage du prolongateur de pointe comprend une première partie (118) et une deuxième partie (120), la première partie ayant un diamètre supérieur à la deuxième partie, la première partie configurée pour recevoir la partie distale de l'endoscope à une partie proximale du prolongateur de pointe, et la deuxième partie configurée pour limiter la translation distale de l'endoscope à l'intérieur du prolongateur de pointe.

12. L'appareil de la revendication 11, dans lequel la deuxième partie de l'alésage est conique et configurée pour fléchir vers l'intérieur afin d'accepter et de retenir un calcul mobile.

13. L'appareil de l'une des revendications 1 à 12, dans lequel le prolongateur de pointe comprend une ou plusieurs rainures (150) s'étendant longitudinalement axialement le long d'une surface extérieure (126) du prolongateur de pointe.

14. L'appareil de la revendication 13, dans lequel le prolongateur de pointe comprend un ou plusieurs passages (158) s'étendant radialement vers l'intérieur entre la ou les rainures et l'alésage.

15. L'appareil de l'une des revendications 1 à 14, dans lequel au moins une partie du prolongateur de pointe est transparente ou translucide.
